# EUROPEAN PATENT SPECIFICATION

(11) **EP 0 319 844 B1**
(45) Date of publication and mention of the grant of the patent: **30.11.1994**
(21) Application number: 88120053.9
(22) Date of filing: 01.12.1988
(51) Int. Cl.: A61B 5/04, H01R 21/22

(54) **Electrical connectors for brain-contact devices**
Elektrische Steckverbindungen für Vorrichtungen mit Kontakt zum Gehirn
Connecteurs électriques pour dispositifs en contact avec le cerveau

(30) Priority: 04.12.1987 US 128797; 13.07.1988 US 218161
(43) Date of publication of application: 14.06.1989
(73) Proprietor: AD-TECH MEDICAL INSTRUMENT CORPORATION, Racine Wisconsin 53402 (US)
(72) Inventor: Putz, David A., Racine Wisconsin 53402 (US)
(74) Representative: Jeck, Anton, Dipl.-Ing.

(56) References cited:
- CA-A- 1 065 969
- FR-A- 2 418 554
- US-A- 3 665 129
- US-A- 3 860 312
- US-A- 4 469 104

## Description

### Field of the Invention

This invention is related generally to electrical connection devices for wire having multiple independent conductive lines and, in particular, to connectors for rapid connection and disconnection. More specifically, this invention is related to multi-electrode brain-contact devices and means on such devices for facilitating surgical procedures for their placement and set-up, including their electrical connection at a position away from the brain.

### Background of the invention

Surgical removal of epileptogenic brain is indicated for treatment of many medically refractory focal seizure disorders. One important factor in providing good results from such surgery is the degree of accuracy in identifying epileptogenic foci. Accurate sensing of intracranial electrical activity, for the purpose of determining epileptogenic foci or otherwise, typically requires use of a plurality of brain contacts. Epileptogenic mapping is one example of the use of electrical devices with plural-contact tissue-engagement members.

Broadly speaking, there are two different kinds of intracranial electrical contact devices - depth probes and flexible flat surface members. Depth probes, often referred to as "depth electrodes", penetrate deep into the brain tissue. One such device is shown in U.S. Patent No. 4,245,645. On the other hand, flexible flat surface members, including those sometimes referred to as "strip" electrodes and "grid" electrodes, are placed subdurally in direct contact with brain tissue at the surface of the brain.

Each of these different kinds of intracranial tissue-engagement members has a plurality of electrodes which are separated from one another by a non-conductive material on which the electrodes are mounted. Separate thin insulated lead wires extend from the tissue-engagement member for each electrode. Such lead wires extend away from the tissue-engagement member to means for connecting the lead wires with individual conductors, which lead to monitoring or recording equipment.

For each type of intracranial tissue-engagement member, the procedures for placement and hookup are of great importance. It is essential that the tissue-engagement members be inserted with a high degree of accuracy in order to avoid damage and in order that placement be in the most advantageous positions. It is also important that the flat flexible member be in proper contact with brain tissue for advantageous results. It is also essential that the lead wires extending from the tissue-engagement member be properly connected and that the fragile lead wires remain functional, without any breakage or disconnection.

While there has been much progress in the field of electrical brain-contact devices in recent years, existing devices and procedures have a number of problems and drawbacks. One significant problem is that the surgical placement and set-up procedures preceding the period of use are far too time-consuming and complex. Such procedures in some cases also lead to specific problems.

Such problems can be described best by generally describing at least certain parts of the placement and set-up procedures, as used, for example, in preparing for an extended period of epileptogenic sensing using grid or strip electrodes as the tissue-engagement members:

One of the early steps in existing placement and set-up procedures for grid and strip electrodes is making an incision in the scalp over the site of proposed electrode placement. Then a burr hole is drilled in the skull or a skull area otherwise removed. One or more incisions are then made in the dura to accommodate placement of a grid or insertion of a strip. Dural tack-up sutures are placed in both dural margins.

The grid or strip electrode device is then placed or inserted, with the electrodes in contact with the brain tissue. When strip electrodes are used, a plurality of strips are usually inserted in each burr hole. The strips and grids may have a large number of electrical contacts. With grid electrodes, the number of contacts may be particularly high. After the grid or strips have been positioned, the dural edges are approximated with a suture.

The lead wires, which extend from the proximal end of each strip or grid, are passed through the sutured dura incision. All the wires, one for each electrode on the grid or on every strip, are then brought out through the skin by passing them through a needle and then drawing them through the scalp at a distance (usually 4-5 cm) from the skull opening. When there are numerous wires it is often necessary to tunnel in a number of directions through the scalp to sites spaced from the skull opening. This can be very time-consuming and very hard on the patient's head.

When such wires have exited the scalp at the chosen sites, it then remains necessary to make electrical hookups of each of such wires in the appropriate manner. This is itself a time-consuming operation, and one in which there is a risk of incorrect hookups. The extended time required for such hookups is a problem in itself. And, the fragile lead wires are quite susceptible to breakage during these manipulative operations; if this occurs, it may be necessary to reopen the dura to remove and replace the grid or strip from which the lead wire broke and repeat many of the procedures described above.

In order to minimize the likelihood of lead wire breakage, lead wires of greater size may be used. However, increasing the diameter of the lead wires tends to increase the overall thickness of the strip or grid. Thickness can be undesirable in such flat flexible members and can in some cases pose problems for the electrical sensing operations. Increasing wire thickness can increase the cost of the device, particularly if silver or platinum wire is used.

When the tissue-engagement member is a depth electrode device, some of the problems may vary to some extent. However, the problems of time-consuming electrical hookup procedures are quite similar. Indeed, such problems are involved with electrical hookup of any plural-contact tissue-engagement member.

There is a substantial need for an improved plural-contact electrical connection device overcoming or minimizing the above problems and difficulties. In particular, there is a need for improved electrical hookup apparatus for use during placement and setup procedures involving electrical contact with brain tissue. A simple plural-contact connector which is easily usable and disposable would be highly desirable.

### Objects of the Invention

It is an object of this invention to provide an improved electrical connector for brain-contact devices overcoming certain problems of the prior art, including those mentioned above.

Another object of this invention is to provide an improved electrical brain-contact device which facilitates surgical procedures used in locating epileptogenic foci.

Another object of this invention is to provide an improved electrical brain-contact device which may be electrically connected easily and quickly during surgical placement and set-up procedures.

Another object of this invention is to provide an electrical connector which reduces or eliminates breakage of lead wires during insertion of brain-contact devices.

Another object of this invention is to provide an improved electrical connection device which provides rapid and accurate electrical hookup of large numbers of electrodes and lead wires during surgical procedures.

Another object of this invention is to provide an improved electrical brain-contact device allowing the use of lead wires of substantially reduced diameter.

Still another object of this invention is to provide an improved electrical connector for use in connecting a plurality of lead wires with individual conductors.

Another object of this invention is to provide an improved electrical brain-contact device facilitating surgical procedures which minimize the infection risks.

Another object is to provide an improved electrical brain-contact device eliminating the need for multiple separate threadings of lead wires in different directions along the scalp, thus eliminating the related scalp damage.

Another object of this invention is to provide an improved electrical connector for plural lead wires which is simple in construction and operation.

These and other important objects will be apparent from the descriptions of this invention which follow.

### Summary of the Invention

The invention is an improvement in electrical devices for brain contact of the type having a tissue-engagement member with a plurality of electrodes, separate lead wires from the tissue-engagement member for each electrode, and means away from the tissue-engagement member to connect the lead wires with individual conductors, such as conductors leading to equipment for recording of electrical impulses. More generally, this invention is an improved electrical connection device of the type for connecting a plurality of lead wires with individual conductors.

In the device of this invention, the lead wires extend to a terminal mount and to an array of lead-wire terminals on and forming a part of the terminal mount. A conductor support holds the individual conductors in a conductor array. A connector block or assembly, hereafter described, serves to join the lead-wire terminals to the individual conductors and hold them firmly in engagement.

The connector block will be described first. Then the alternative connector assembly (sometimes referred to the "connector") will be described.

The connector block has a first space and an array of second spaces each of which intersect the first space. The first space receives the terminal mount and the array of second spaces receives the conductor array. Broadly described, the connector block, terminal mount and conductor array are configured and arranged such that the lead-wire terminals and individual conductors are held in engagement by mechanical interference.

The terminal mount is an elongate member having the lead-wire terminals spaced therealong. For example, the terminal mount may include a tubular sheathing covering all the lead wires and rings around and spaced along the sheathing forming the lead-wire terminals. In such cases, the first space in the connector block may be a first elongate cavity in the connector block formed of non-conductive material and shaped to receive the elongate member.

In such preferred embodiment, the individual conductors are preferably male members which protrude from the conductor support. The second spaces are preferably second elongate cavities in the conductor block formed of non-conductive material and shaped to receive the male members.

Preferred embodiments of this invention include means biasing the male members and lead-wire terminals together at the intersections of the second elongate cavities with the first elongate cavity. In one form of such preferred embodiment, the lead-wire terminals have substantially fixed cross-dimensions, the connector block has a pair of adjacent second elongate cavities corresponding to each lead-wire terminal, and each individual conductor includes a pair of said male members which enter the pair of second elongate cavities and spread slightly to receive a lead-wire terminal.

The male members of each pair of male members are spaced from each each other by a distance less than the fixed cross-dimension of the corresponding lead-wire terminal. This dimensioning requires forcible spreading of the male members of each pair for engagement with the corresponding lead-wire terminal. The forcible spreading provides the biasing means for reliable electrical contact and mechanical engagement.

The connector block is preferably substantially transparent. This may be accomplished by molding or machining the connector block of a tough substantially transparent plastic material. Using such materials, allows viewing of the engagement of the conductors with the lead-wire terminals, which provides assurance of proper connection. The connector block is preferably integrally formed of just one material. Each block is preferably of non-conductive one-piece construction.

The connector block of this invention preferably includes a main surface which extends substantially parallel to the first elongate cavity, with the second elongate cavities each extending from such main surface to intersection with the first elongate cavity. The connector block may be a single block with any number of second elongate cavities. Or, the connector block may be formed of a plurality of sub-blocks which are attached end-to-end by mating means on each adjacent pair of sub-blocks.

In the case of plural sub-blocks, each sub-block forms a portion of the first elongate cavity and a portion of the main surface. And each sub-block has a subset of the second elongate cavities. Thus, the connector block can be sized to accommodate varying numbers of lead-wire terminals and conductors by choosing the number of sub-blocks to be attached to one another.

The mating means which join the sub-blocks preferably have stop means facilitating co-alignment of the first elongate cavity portions and co-alignment of the main surface portions. The mating means holding the sub-blocks together are preferably corresponding male and female dove-tail connectors, and the stop means used for alignment purposes may be the ends of the female and male elements.

In certain preferred embodiments, the main surface of the connector block includes an off-center recess (hole) on it which serves to dictate the orientation of the conductor support with respect to it when the conductors extending therefrom are engaged in the second elongate cavities. A guide pin, which extends from the conductor support along with the male conductor members, is received in the off-center recess only when the conductor support is in the proper orientation. This serves as a further means to assure accurate connection of the conductor members to the lead-wire terminals.

In some preferred embodiments, the connector block has opposed finger-grip protrusions along its opposed sidewalls. Such protrusions serve to facilitate detachment of the connector block from the conductor support, and, when a sub-block construction is used, to aid in detachment of the sub-blocks from one another.

In certain embodiments of this invention, as noted above, a connector assembly is used instead of the connector block described above. Such connector assembly includes a base member with an array of take-up terminals matching the array of lead-wire terminals on the terminal mount, a yoke member which is movable with respect to the base member, and means to hold the yoke and base members together with the terminal mount between. This secures the array of lead-wire terminals and conductor array in terminal-to-terminal contact. The base and yoke members together form a space for the terminal mount which is equivalent to the first space of the conductor block described above.

In certain preferred forms, the base member of the connector assembly forms terminal niches along the terminal-mount space, with the take-up terminals being within such niches, most preferably extending, when the terminal mount is removed, from the niches into the terminal-mount space. In certain embodiments, the take-up terminals are preferably spring members, such as small coil springs, which are depressed by the terminal mount when the terminal mount is in place. This preferred arrangement holds the lead-wire terminals and the take-up terminals in firm electrical contact.

In certain preferred embodiments, the base and yoke members have major portions which are semi-cylindroids together forming a cylindroid, and the holding means is a clamp ring extending around the cylindroid. In such configuration, the space for the terminal mount is most preferably along the axis of the cylindroid.

The clamp ring may be in various forms. In one form, the clamp ring has a gap in it large enough such that the lead wires can pass through the gap to facilitate mounting of the clamp ring on the cylindroid. The clamp ring may readily be brought into axial alignment with the cylindroid by virtue of such gap, and then slid over the cylindroid. The dimensioning and resilient characteristics of the ring allow it to hold the semi-cylindroids firmly together.

In particularly preferred embodiments of the type just described, either the base member or the yoke member has brackets at either end, while the other of such members is dimensioned to be received between such brackets. One of the brackets forms an axial opening receiving the terminal mount, which in such embodiments has tubular sheathing with electrode rings on it.

The tubular sheathing preferably has a marking on it which is alignable with an end bracket, preferably the bracket having the axial opening mentioned above, to facilitate alignment of the lead-wire array with the take-up array. The other bracket may form a stop for the tubular sheathing as an alternate or additional way to facilitate alignment of the terminal arrays. Such other bracket may have its own axial opening to receive and hold the terminal mount in proper position along the axis of the cylindroid. This serves to facilitate proper assembly of the terminal mount with the connector means.

In certain other very highly preferred embodiments of this invention, the base and the yoke of the connector assembly are in a different form having additional significant advantages. In such embodiments, the base is a first block having a first surface and the yoke is a second block having a second surface against the first surface, with at least one of the first and second surfaces having a major groove which forms the space for the terminal mount. The take-up terminals are secured to the first block and extend in spaced fashion across the major groove.

In such embodiments, the base and yoke blocks are preferably held together by one or more screw members, preferably one as later explained, extending between the first and second blocks. Such screw members, which may be completely removably to allow complete removal of the second block from the first if desired, are used to progressively draw the first and second surfaces of such blocks together. The screws are preferably finger-operable.

In preferred embodiments of the types having base and yoke blocks, the major groove is preferably in the first surface, that is, in the base (or first) block. The second surface is preferably flat, with the terminal mount, major groove, and take-up terminals dimensioned and arranged such that drawing the first and second surfaces together clamps the arrays firmly together. More specifically, the depth of the major groove is small enough that, when the base and yoke blocks are drawn tightly together, the terminal mount is tightly sandwiched between such blocks.

In such embodiments of the invention, there is preferably only one screw member which serves as the sole interconnection of the base and yoke blocks. Such screw member may extend through the yoke (or second) block freely and be in threaded engagement with the base (or first) block. In such embodiments, the screw member may be loosened short of disengagement with the base block such that the yoke block may be pivoted with respect to the base block to substantially uncover the major groove. This facilitates insertion of the terminal mount when the screw member is loosened. Indeed, loosening and retightening to insert the terminal mount, preferably by finger-turning of the screw member, can be quickly carried out, and with one hand if necessary.

In embodiments of the type with base and yoke blocks, as described, the base block has a side surface parallel to the major groove and the take-up terminals are preferably straight wires which are embedded within the base block, extend across the major groove, and terminate in connector ends exposed along the side surface. This facilitates organized electrical connection with the device of this invention, whether by individual connectors or by a multi-contact plug.

One of the blocks, preferably the base (or first) block, may be marked with colors, numbers, letters or other indicia to identify which contact relates to which electrode in use during a test period.

The base block preferably has one open end and one closed end for the major groove. This facilitates proper alignment of the contact arrays.

Referring again to characteristics of the terminal mount, earlier described, the preferred tubular sheathing which forms the terminal mount preferably has a tapered lead end. Such tapered lead end facilitates various insertion steps, including in some cases insertion through a needle, for purposes hereafter explained, as well as insertion into the connector block or connector assembly and over the take-up terminals as the sheathing moves into the connector block or assembly.

In certain preferred embodiments, a pull line is attached to the terminal mount, such as by connection to the inside of the sheathing forming such terminal mount, and extends from the terminal mount in a direction away from the lead wires. This further facilitates threading of the terminal mount and lead wires through a needle and into the aforementioned connector block or assembly, making surgical procedures that much easier.

### Brief Description of the Drawings

FIGURE 1 is an enlarged perspective view of a preferred electrical brain-contact device in accordance with this invention, with its electrical connection device assembled as in use.
FIGURE 2 is a reduced fragmentary perspective view of the device of FIGURE 1, showing the electrical connection device in unconnected condition.
FIGURE 3 is an enlarged sectional end view of FIGURE 1, taken along section 3-3 as indicated in FIGURE 1.
FIGURE 4 is a fragmentary top sectional view of the device of FIGURE 3, taken along section 4-4 as indicated in FIGURE 3.
FIGURE 5 is an enlarged perspective view of another embodiment, which has a plurality of sub-blocks.
FIGURE 6 is a fragmentary side sectional view of the device of FIGURE 5, taken along section 6-6 as indicated in FIGURE 5.
FIGURE 7 is perspective view of a electrical depth probe device in accordance with this invention.
FIGURE 8 is an enlarged fragmentary side elevation of the device of FIGURE 7 with the connector removed and a stellate inserted.
FIGURE 9 is an enlarged fragmentary sectional view of the device shown in FIGURES 7 and 8, a portion thereof which is insertable into the connector.
FIGURE 10 is perspective view of a grid electrode in device in accordance with this invention.
FIGURE 11 is an exploded perspective of the connector included in the embodiments of FIGURES 7 and 10, for which the connectors are identical.
FIGURE 12 is a side elevation of such connector.
FIGURE 13 is a side elevation of the base member of such connector, with portions cut away.
FIGURE 14 is a perspective view, similar to FIGURE 7, of another embodiment, having another connector in accordance with this invention.
FIGURE 15 is a fragmentary view of the device of FIGURE 14, but showing the terminal mount inserted into the connector.
FIGURE 16 is an exploded view of the connector of FIGURE 14.
FIGURE 17 is a partially cutaway plan view of the connector of FIGURE 14.
FIGURE 18 is a partially cutaway end view of the connector of FIGURE 14.

### Detailed Descriptions of Preferred Embodiments

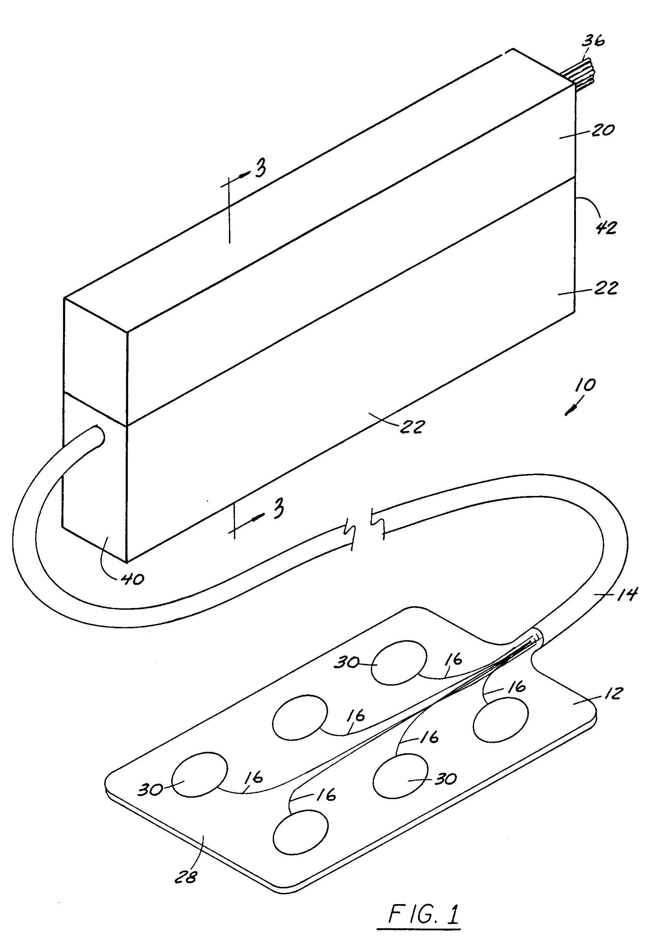

FIGURES 1-6 and 7-18 illustrate various forms of two embodiments of this invention. These groups of drawings will be described separately.

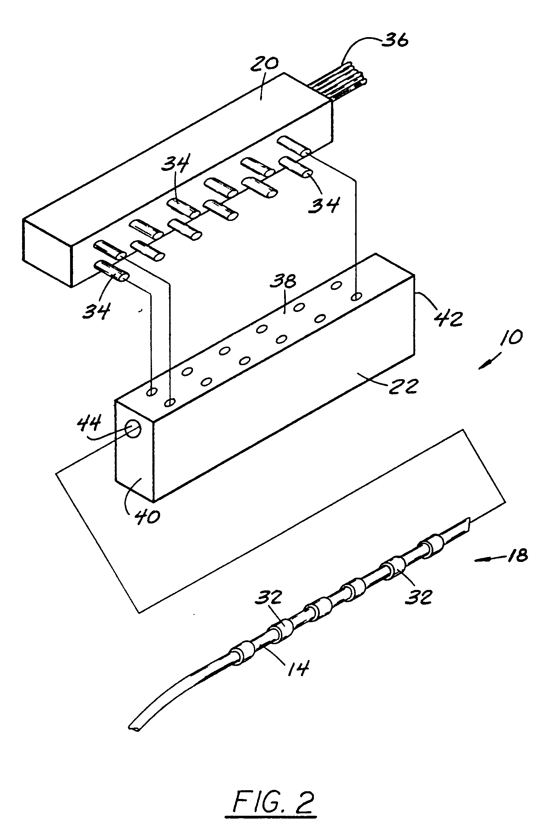

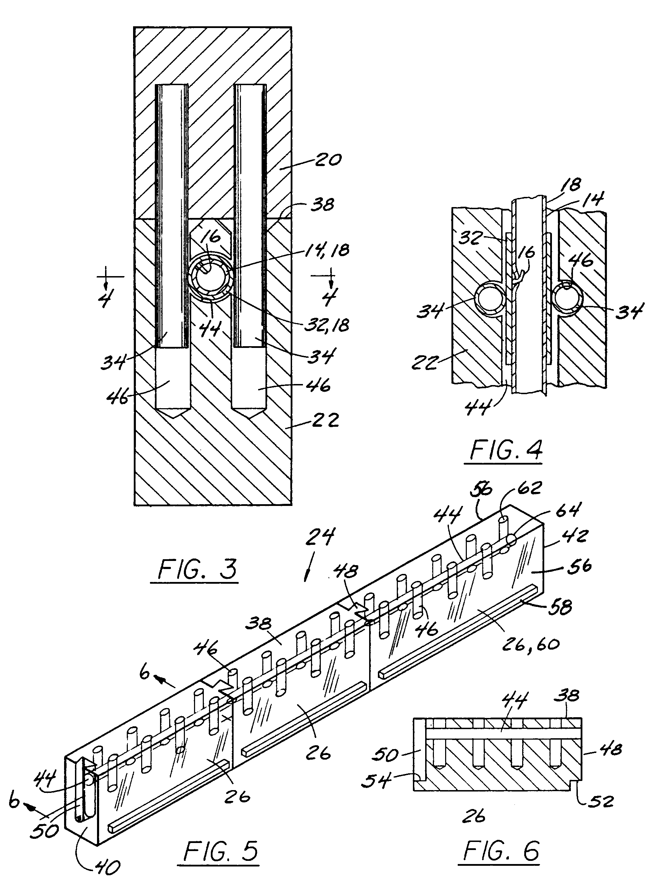

Turning first to the FIGURE 1-6 embodiments, FIGURES 1-4 illustrate an electrical brain contact device 10 including a tissue-engagement member 12, a tubular sheathing 14 within which lead wires 16 extend toward a terminal mount 18 (see FIGURE 2), a conductor support 20, and a single unitary connector block 22. FIGURES 5 and 6 illustrate an alternative connector block 24 formed of a plurality of sub-blocks 26. Like parts are identified by like numerals in FIGURES 1-6.

Tissue-engagement member 12, shown in FIGURE 1, is a "grid electrode" which includes a flexible sheet member 28 and a number of flat electrode disks 30 held by and coplanar with flexible sheet member 28. Sheet member 28 is formed by a pair of flexible sheets with electrode disks 30 between them. Also held between the flexible sheets of sheet member 28 are lead wires 16, one wire attached to each electrode disk 30. Lead wires 16, each of which has its own thin layer of insulation, come together at the edge of flexible sheet member 28 where they enter sheathing 14.

Lead wires 16 and sheathing 14 over them extend from tissue-engagement member 12 to terminal mount 18, shown best in FIGURE 2. Terminal mount 18 is an elongate member. Sheathing 14 extends into and becomes part of terminal mount 18, forming a base for mounting of lead-wire terminal rings 32. Each lead wire 16 comes through sheathing 14 at the a lead-wire terminal ring 32 and is electrically connected to such ring at a position between ring 32 and sheathing 14. Lead-wire terminal rings 32 are spaced along terminal mount 18 and form an array of lead-wire terminals.

Conductor support 20 holds several pairs of male conductor members 34 in an array. The spacing between adjacent pairs of male conductor members 34 is equal to the spacing between adjacent lead-wire terminal rings 32. Conductor support 20 is a non-conductive substantially rigid member. The male conductor members of each pair of conductor members are electrically joined and lead to an individual wire 36 which may be connected, for example, to electrical sensing and reporting apparatus not shown.

Connector block 22 serves as a convenient disposable means for connecting male conductor members 34 to lead-wire terminal rings 32. Connector block 22 is an integrally formed unitary non-conductive block which has a main surface 38 and first and second ends 40 and 42.

A first elongate cavity or space 44 and an array of second elongate cavities or spaces 46 are formed in connector block 22. First elongate cavity 44 is parallel to main surface 38 and extends the full length of connector block 22. First elongate cavity 44 is complementary to terminal mount 18; that is, first cavity 44 is a generally cylindrical void which receives terminal mount 18. Second elongate cavities 46 each intersect first cavity 44, as illustrated best in FIGURES 3 and 4. Second cavities 46 are complementary to male connector members 34, allowing insertion thereof with a slight degree of freedom.

A pair of second cavities 46 accommodates each pair of male conductor members 34. Each pair of second cavities 46 is spaced from its adjacent pair of second cavities by a distance equal to the spacing between adjacent of lead-wire terminal rings 32 and to the spacing between adjacent pairs of male conductor members 34. Thus, when terminal mount 18 is inserted into first elongate cavity 44, male conductor members 34 may be inserted into second elongate cavities 46 to contact lead-wire terminal rings 32 at each position of insertion of male conductor members.

In use, with terminal mount 18 inserted in first elongate cavity 44, conductor support 20 is brought toward connector block 22 until male conductor members 34 are inserted in second elongate cavities 46. Connector block 22, first cavity 44, second cavities 46, conductor support 20 and its lead-wire terminal rings 32, and male conductor members 34 are configured and arranged such that lead-wire terminal rings 32 and male conductor members 34 are held in engagement by mechanical interference.

More specifically, lead-wire terminal rings 32 have a fixed cross-dimension and male conductor members 34 of each pair are spaced from one another by an amount less than such fixed cross-dimension. By this configuration, such pair of male conductor members 34 are forcibly spread when engaging lead-wire terminal ring 32 therebetween at the intersection of second cavities 46 with first cavity 44. Such spreading causes male conductor members 34 to be biased to a return position such that they squeeze lead-wire terminal ring 32 therebetween to provide firm electrical contact.

As illustrated in FIGURES 5 and 6, an alternative to the one-piece connector block of FIGURES 1-4 is connector block 24 of FIGURES 5 and 6 which is formed of a plurality of sub-blocks 26. Sub-blocks 26 are attached end-to-end by corresponding male and female dovetail connectors on each adjacent pair of sub-blocks 26. Each sub-block 26 forms a portion of a first elongate cavity 44, such portions being aligned. Each sub-block 26 also forms a portion of main surface 38, such portions being coplanar.

Male and female dovetail connectors 48 and 50 have ends 52 and 54, respectively, which form stop means facilitating co-alignment of first elongate cavity portions and co-alignment of the main surface portions when sub-blocks 26 are assembled.

The use of sub-blocks 26 allows connector block 24 to be sized to accommodate any number of lead-wire terminals and individual conductors. The appropriate number of sub-blocks 26 is chosen and they are connected by means of male and female connectors 48 and 50. Then terminal mount 18, regardless of its length and number of terminal rings 32, is inserted into first elongate cavity 44 formed by interconnected sub-blocks 26, and conductor support 20 with a corresponding number of pairs of male conductor members 34 is inserted into second elongate cavities 46 to cause electrical connection of corresponding pairs of terminal rings 32 and male conductor members 34.

Extending from the opposed sidewalls 56 of sub-blocks 26, near the edges thereof farthest from main surfaces 38, are finger-grip protrusions 58. Such protrusions serve to facilitate detachment of connector block 24 from the conductors used therewith, and also aid in detachment of one sub-block from another.

One of the sub-blocks 26 is an end block 60. End block 60 is somewhat longer than the other sub-blocks. End block 60 provides a closed end 64 for first elongate cavity 44 at second end 42 of connector block 24. End block 60 also includes an off-center recess 62 on the connector block main surface 38, shaped like one of second elongate cavities 46. Off-center recess 62 serves to dictate the orientation of conductor support with respect thereto, as explained above.

Using either a one-piece unitary connector block 22 or a connector block 24 formed of sub-blocks 26 greatly facilitates connection of lead wires from tissue-engagement members of brain-contact devices. This substantially shortens the length of a difficult surgical procedure and eliminates any possibility of connection error.

The device of FIGURES 1-6 may be made using a variety of readily available parts and materials. Flexible sheet member 28 is preferably a dielectric silicone sheet material such as SILASTIC sheeting, a material available from Dow Chemical Company, Midland, Michigan USA. Tubular sheathing 14 is preferably made of SILASTIC or of a polyurethane member such as TECOFLEX available from Thermedics, Inc., Woburn, Massachusetts USA. A variety of other suitable non-conductive materials may be used for sheathing 14.

A variety of conductive materials may be used for lead-wire terminal rings 32. Lead-wires 16 are preferably stainless steel, platinum, or silver strands insulated by a teflon coating layer. Connector block 22 and sub-blocks 26 are preferably formed by a molding or machining process using a polysulfone or polycarbonate resin. A preferred material is the polycarbonate resin known as LEXAN, available from General Electric, Schenectady, New York USA.

In certain highly preferred embodiments, blocks 22 and 26 are substantially transparent, as is possible when using, for example, LEXAN. Use of a substantially transparent material allows observation of the engagement of male conductor members 34 with terminal rings 32.

As noted, blocks 22 and sub-blocks 26 are preferably of unitary construction, being entirely free of conductive material, particularly on the walls of cavities 44 and 46. Blocks 22 and 24 are simple devices and are intended to be discarded after use.

Many variations are possible in the devices of FIGURES 1-6. For example, a variety of tissue-engagement members may be used, as noted above, including depth electrodes, strip electrodes and grid electrodes (as shown). Likewise, a variety of conductor supports may be used. Considerable variation is possible in connector block 22 and 24, including variations in shape and variations in the nature of the mating means hold sub-blocks 26 together.

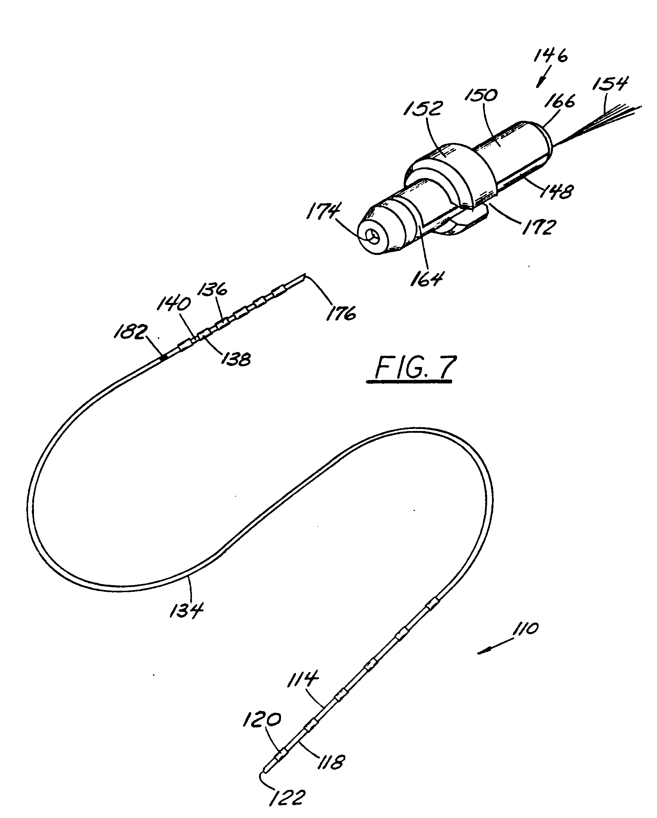

Turning now to the embodiments of FIGURES 7-18, such drawings illustrate certain preferred forms of the electrical brain-contact device of this invention having various tissue engagement members and various connector assemblies in accordance with this invention. Throughout these figures, like parts are identified by like numbers. In some cases, comparable parts are identified by like numerals followed by the letter "a."

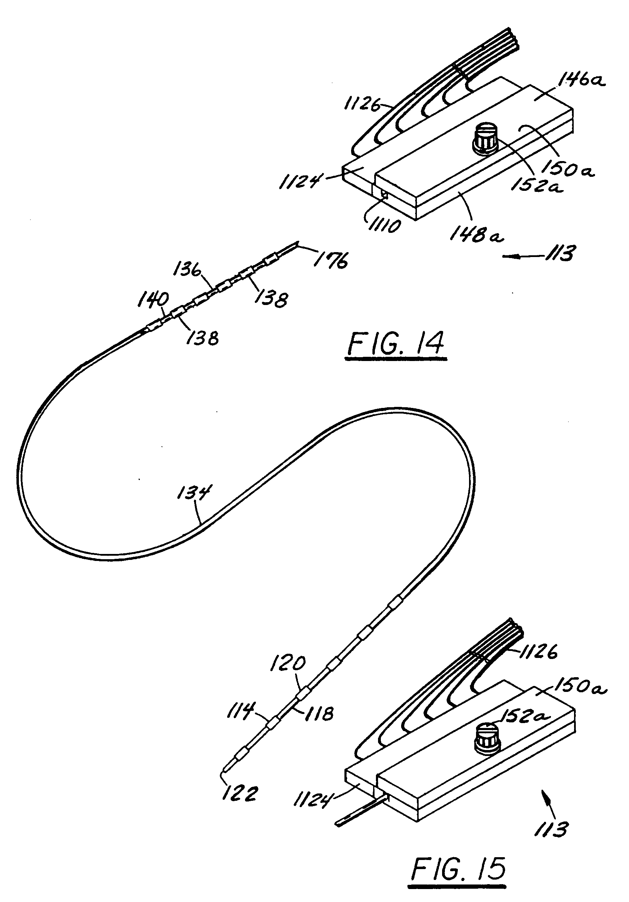

Three embodiments are illustrated in FIGURES 7-18, including a depth probe device 110 shown in FIGURES 7-9 and 11-13, a surface contact device 112 shown in FIGURES 10, 9 and 11-13, and another depth probe device 113 shown in FIGURES 14-18, 8 and 9. Devices 110 and 112 have identical connector assemblies 146 in accordance with this invention, while device 113 has a connector assembly 146a which is a variation.

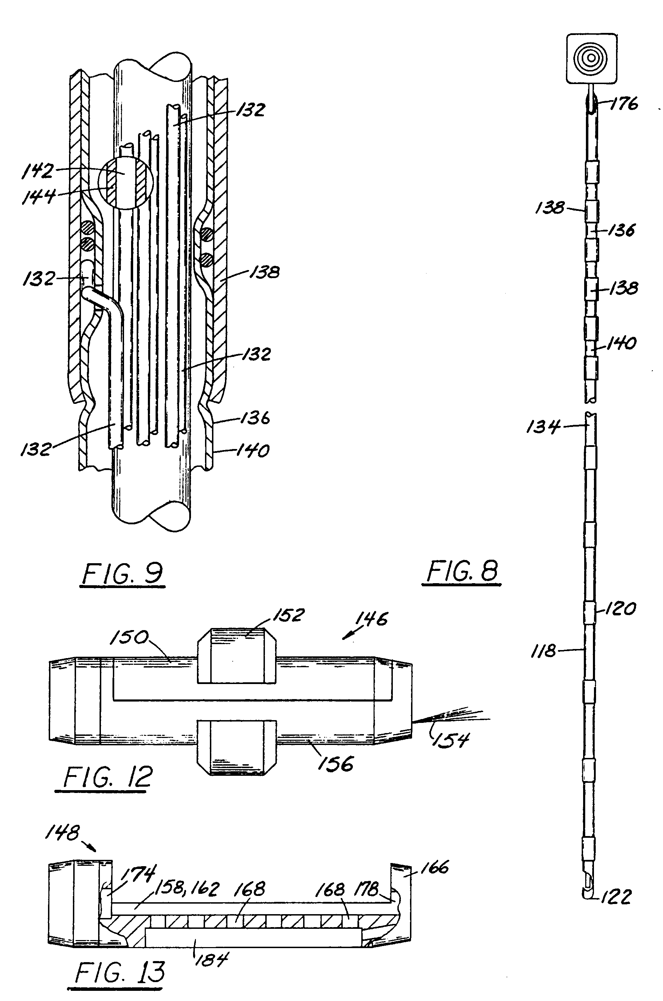

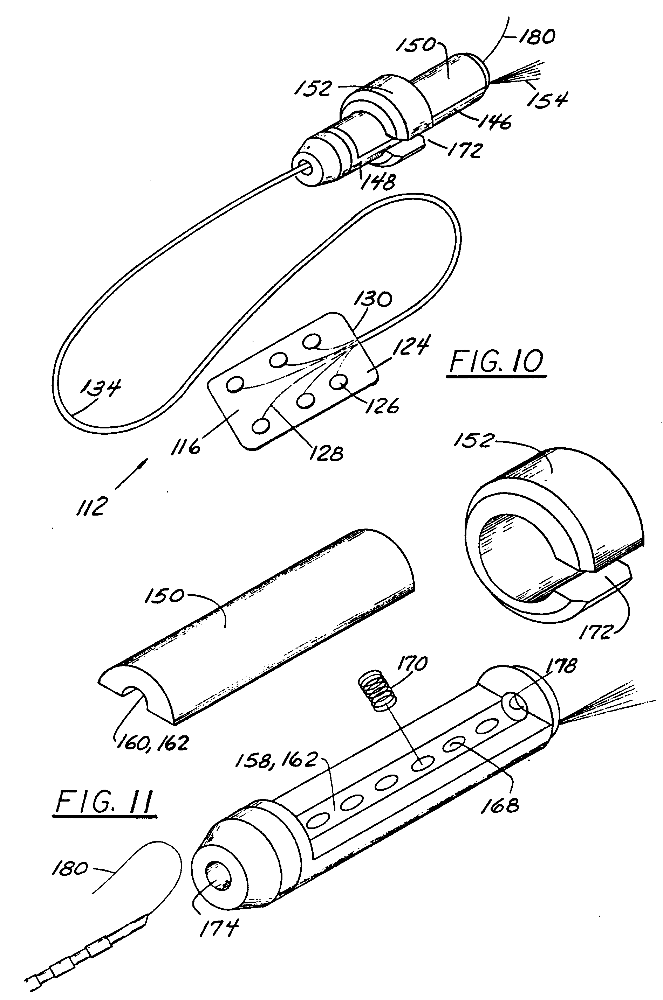

Devices 110, 112 and 113 all include identical terminal mounts 136, shown in FIGURES 7-9, 11 (in fragment) and 14. The details of terminal mounts 136 are shown best in FIGURES 8 and 9.

Devices 110 and 113 have identical tissue-engagement members, that is, depth probes 114, while surface-contact device 112 has a different tissue-engagement member, namely, flat flexible member 116, sometimes referred to as a "grid electrode." Flat flexible member 116 may be part of a device having a connector like connector 146a, although that is not specifically shown. Depth probes 114 and flat flexible member 116 each place a plurality of electrodes directly in contact with brain cells, to sense electrical discharges.

Depth probe 114 includes a non-conductive hollow plastic tube 118 having electrode rings (or "collars") 120 spaced along it and attached to it. Hollow tube 118 has a closed distal end 122. Individual lead wires extend inside hollow tube 118 from each electrode ring 120 in a direction away from distal end 122. Depth probe 114 itself is of a type known in the prior art.

Flat flexible member 116 includes a flexible sheet member 124, which is formed by a pair of flexible sheets, and a number of flat electrode disks 126 on and coplanar with sheet member 124. Electrode disks 126 are held on sheet member 124 by being placed between its two layers. Individual wires 128 are connected with each electrode 126 and extend toward a proximal edge 130 of flat flexible member 116. Flat flexible member 116 is similar to prior grid and strip electrodes, except that it may be substantially thinner than prior grids and strips.

The wires, such as wires 128, which are within the different types of tissue-engagement members illustrated in the drawings exit such tissue-engagement members as lead wires 132. Lead wires 132 extend from either depth probe 114 or flat flexible member 116 through tubular sheathing 134 to a terminal mount 136. More specifically, lead wires 132 extend to an array of lead-wire terminal rings 138 which form a part of terminal mount 136.

In each of the illustrated embodiments of FIGURES 7-18, terminal mount 136 includes tubular sheathing 140 which forms a base for mounting of lead-wire terminal rings 138. Lead wires 132 extend through tubular sheathing 140 in the manner shown in FIGURE 9, with each lead wire 132 attached to one lead-wire terminal ring 138.

Tubular sheathing 140, tubular sheathing 134, and hollow tube 118 are all formed of the same tube stock. Such tubing is preferably made of silicone or polyurethane materials, such as SILASTIC or TECOFLEX, mentioned above, or of other suitable non-conductive materials. Sheet member 124, which forms part of flat flexible member 116, is preferably made of SILASTIC sheeting, mentioned above.

The lead wires extending along and from depth probe 114 or sheet member 124 to and along terminal mount 136 are preferably stainless steel, platinum or silver strands 142 which are insulated by a teflon coating layer 144. See FIGURE 9. Such wires, although fragile in themselves, are protected by tubular sheathing 134 and 140 around them. Since they are not individually manipulated such wires are not particularly susceptible to breakage.

Because of the protection of such sheathing, the lead wires may be made much smaller than lead wires in devices of the prior art. In such prior devices, wire has typically been no less than about 0.38 mm in diameter. Given the protection of tubular sheathing 134 and tubular sheathing 140, wires 128 can be as small as about 0.125 mm in diameter. This allows the flexible member to have a thickness of less than about 0.30 mm, making flat flexible member 116 thinner than previous strip and grid electrodes.

As illustrated in FIGURES 7 and 10-13, each of the illustrated brain-contact devices has a connector 146 to which a terminal mount 136 is joined. Connector 146 includes a base member 148, a yoke member 150, which is movable with respect to base member 148, a clamp ring 152, and outlet wires 154 which extend toward connection with monitoring apparatus or the like. Outlet wires 154 are quite strong in comparison with any of the lead wires; they may be freely manipulated and connected as desired without much risk of breakage.

All of yoke member 150 and a major portion 156 of base member 148 are semi-cylindroids which together form a single cylindroid when connector 146 is assembled. Base member 148 and yoke member 150 form first and second semi-cylindrical grooves 158 and 160 which extend along the axis of the cylindroid. First and second semi-cylindrical grooves 158 and 160 together form cylindrical terminal-mount space 162 which is sized to receive terminal mount 136 when terminal 136 and connector 146 are joined.

Base member 148 has first and second brackets 164 and 166 at opposite ends of its semi-cylindroid portion 156. The spacing of first and second brackets 164 and 166 is such that yoke member 150 fits snugly between such brackets when connector 146 is assembled.

A number of terminal niches 168 are formed in base member 148 in positions spaced along first semi-cylindrical groove 158. Niches 168 are cylindrical and are transverse to the axis of connector 146. Located in each niche 168 is a small coil spring 170. Springs 170 are take-up terminals which extend from niches 168 into terminal-mount space 162 when terminal mount 136 is not present.

Each outlet wire 154 is electrically connected to one and only one take-up terminal 170 at a position in or near the terminal niche 168 containing such terminal 170. Outlet wires 154 extend to take-up terminals 170 through an outward groove 184 which is along the wall of base member 148 at a position adjacent to niches 168. Outlet wires 154 extend through an end opening in second bracket 166, and epoxy within outward groove 184 serves to hold outlet wires 154 and take-up terminal springs 170 in place.

Take-up terminal springs 170 are in an array which matches the array of lead-wire terminals on terminal mount 136. When terminal mount 136 is in place in terminal-mount space 162, such arrays are in terminal-to-terminal contact. When yoke member 150 is assembled with base member 148, terminal rings 138 of terminal mount 136 depress take-up terminal springs 170 toward their respective niches 168. This provides firm electrical contact between take-up terminals 170 and lead-wire terminals 138.

During assembly of yoke member 150 with base member 148, clamp ring 152 is brought into a position of axial alignment with the remainder of connector 146 beyond the end thereof and is then slid over base member 148 and yoke member 150. Clamp ring 152 has a gap 172 in it which is big enough for wires to pass as clamp ring 152 is brought into its position of alignment. The dimensions and resiliency characteristics of clamp ring 152 are such that ring 152 will hold yoke member 150 tightly in place against base member 148. This serves to assure that terminal mount 136 remains properly in place.

Insertion of terminal mount 136 into terminal-mount space 162 requires its insertion through an axial opening 174 in first bracket 164. Such insertion is facilitated by means of tapered lead end 176 of terminal mount 136. Such tapering also facilitates the movement of terminal mount 136 over take-up terminal springs 170 and the insertion of the end of terminal mount 136 into an axial opening 178 in second-bracket 166.

Second bracket 166 itself functions as a stop for engagement by tubular sheathing 140 of terminal mount 136 to facilitate alignment of terminal arrays. Second-bracket axial opening 178 serves to hold terminal mount 136 in the proper position, along the axis, thus facilitating proper assembly of terminal mount 136 with connector 146.

Tapered lead end 176 also facilitates insertion of terminal mount 136 through a needle. This is particularly useful in placement and set-up of electrical brain-contact devices having flat flexible members, the lead wires of which are preferably threaded away from the position of the skull opening.

As illustrated in FIGURES 10 and 11, a pull line 180 is attached to terminal mount 136 inside tubular sheathing 140 and extends away from terminal mount 136 in a direction away from its lead wires. Such pull line, which is preferably a flexible, insulated multi-stranded metallic wire of somewhat heavier gauge than the lead wires, is another means to facilitate the threading of terminal mount 136 and tubular sheathing through a needle, or for that matter through axial openings 174 and 178. Pull line 180, which is small in dimension compared to terminal mount 136 and tubular sheathing 134, is first threaded through the needle, or axial opening, and then used to pull terminal mount 136 and tubular sheathing 134 therethrough.

While alignment of the terminal arrays may be accomplished visually or by means of a stop member such as second bracket 166, alignment may also be facilitated by the presence of a marking 182 on tubular sheathing 140. Marking 182, which may be a numbered and/or colored band or suitable marking of any other kind, is located in a position such that its alignment with the end of terminal mount 136 or with some marking on terminal mount 136 causes proper alignment of the terminal arrays.

Turning now to a more detailed description of FIGURES 14-18, connector 146a of depth probe device 113 includes a base block 148a (sometimes referred to herein as first block 148a), a yoke block 150a (sometimes referred to herein as second block 150a), which is movable with respect to base block 148a, and a finger screw 152a. Base block 148a and yoke block 150a are flat rectangular blocks which are drawn together and held together by finger screw 152a.

First or base block 148a and second or yoke block 150a have first and second surfaces 1102 and 1104, respectively, which are facing each other. Second surface 1104 is against first surface 1102. Yoke block 150a has a hole through it, in a generally central position, which freely receives the shank 1106 of finger screw 152a. Base block 148a has a threaded opening which is engaged by shank 1106, which is threaded. Adjustment of finger screw 152a tightens and loosens the relationship of blocks 148a and 150a and also allows complete removal of block 150a. Finger screw 152a has a head which allows easy finger adjustment.

First surface 1102 of base block 148a has a major groove 158a in it extending from an open end 1110 at one end of base block 148a to a closed end 1112 near the opposite end of base block 148a. Major groove 158a, itself in the shape of a rectangular block, forms a terminal-mount space 162a which is sized to receive terminal mount 136 when terminal 136 and connector 146a are joined.

Second surface 1104, which adjoins major groove 158a, is flat, and forms a boundary for terminal-mount space 162a. Second surface 1104 could, however, have a groove adjacent to the groove in first surface 1102; in such case the grooves would together form the terminal-mount space. However, having second surface 1104 flat is preferred for ease of use. Surfaces 1102 and 1104 may have small mating male and female features which, when the blocks are drawn together, tend to hold them in proper alignment.

For each lead-wire terminal ring 138 of terminal mount 136, base block 148a has a take-up terminal 170a which is secured to the block and extends across major groove 158a. Take-up terminals 170a are fairly rigid wires spaced along major groove 158a in an arrangement corresponding to the spacing of terminal rings 138 along terminal mount 136. Take-up terminal wires 170a are of diameter significantly less than the depth of major groove 158a.

For each wire 170a, there is a shallow transverse groove 168a in the bottom of major groove 158a, with the wire lying in it. The depth of transverse grooves 168a are much less than the diameters of wires 170a, such that wires 170a extend well above the bottom of major groove 158a.

Terminal mount 136, the depth of major groove 158a, and take-up terminal wires 170a are dimensioned and arranged such that bringing first and second surfaces 1102 and 1104 of blocks 148a and 150a together clamps the array of lead-wire terminals 138 firmly against the corresponding array of take-up terminals 170a.

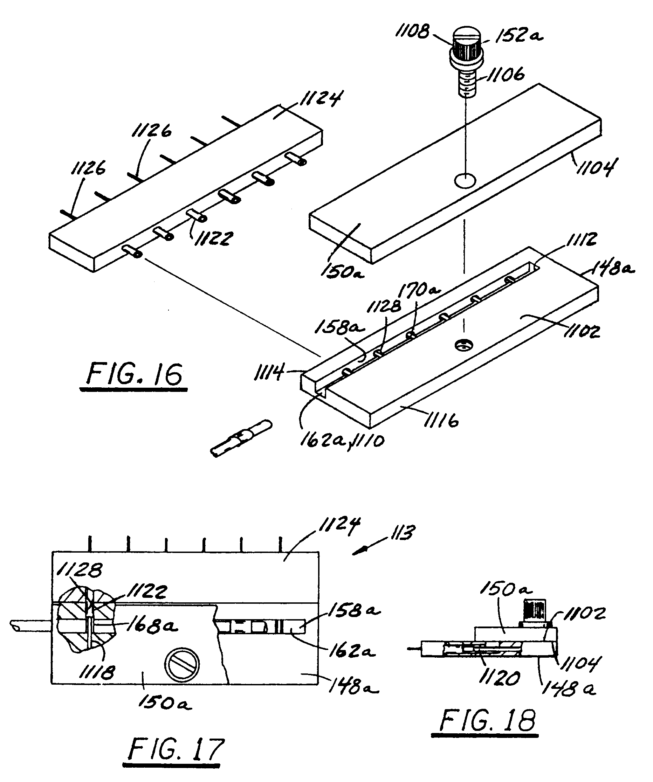

First or base block 148a has first and second side surfaces 1114 and 1116 which are parallel to major groove 158a. Take-up terminals 170a are straight wires which have first end portions 1118 (see FIGURE 17) embedded in base block 148a in the area between major groove 158a and second side surface 1116. Take-up terminal wires 170a are exposed, as indicated, along major groove 158a, and extend on to terminate at connector ends 1120 which are exposed along first side surface 1114.

Connector ends 1120 are held in concentric fashion within small cylindrical openings 1128 spaced along first side surface 1114 in position to receive annular prongs 1122 of a hook-up bar 1124 from which wires 1126 extend to monitoring equipment. Instead of hook-up bar 1124, individual wires can be connected to connector ends 1120.

When terminal mount 136 is inserted into major groove 158a, its end comes into engagement with closed end 1112 of groove 158a. When this occurs, lead-wire terminal rings 138 are in proper alignment with take-up terminals 170a. This further facilitates connection.

The improved electrical brain-contact devices of this invention may be made using well-known materials and construction techniques. Acceptable materials and techniques would be apparent to those skilled in the art who are familiar with this invention.

Many variations are possible. For example, the connectors can vary significantly as long as the connector and terminal mount are adapted for each other. Strips may be used instead of the grids and depth probes which are illustrated. Large grids can have more than one grouping of lead wires within a sheathing, with a separate connector for each grouping. A great number of variations may be made in many of the parts and structures of this invention.

While the principles of this invention have been described in connection with specific embodiments, it should be understood clearly that these descriptions are made only by way of example and are not intended to limit the scope of the invention.

## Claims

1. An electrical brain-contact device (10,110,112,113) having a tissue-engagement member (12,114,116) with a plurality of electrodes (30,120,126), separate lead wires (16,132) from the tissue-engagement member (12,114,116) for each electrode (30,120,126), and means (20,22,24,146,146a) away from the tissue-engagement member (12,114,116) to connect the lead wires (16,132) with individual conductors (34,170,170a),
characterized by:
- the lead wires (16) extending to a terminal mount (18) and to an array of lead-wire terminals (32) on and forming a part of the terminal mount (18), the terminal mount (18) including a sheathing (14) with the lead wires (16) therein and the lead-wire terminals (32) spaced therealong;
- the connector means including a conductor support (20) holding the individual conductors (34) in a conductor array; and
- a connector block (22,24) having a first space (44) and an array of second spaces (46) each intersecting the first space (44), the first space (44) receiving the terminal mount (18) and the array of second spaces (46) receiving the conductor array, the connector block (22,24), terminal mount (18) and conductor array configured and arranged such that the lead-wire terminals (32) and individual conductors (34) are held in engagement by mechanical interference.

2. The electrical brain-contact device (10) of claim 1 characterized in that:
- the terminal mount is an elongate member (18) having the lead-wire terminals (32) spaced therealong;
- the first space is a first elongate cavity (44) in the connector block (22,24) formed of non-conductive material and shaped to receive the elongate member (18);
- the individual conductors are male members (34) protruding from the conductor support (20); and
- the second spaces are second elongate cavities (46) in the connector block (22,24) formed of non-conductive material and shaped to receive the male members (34).

3. The electrical brain-contact device (10) of claim 2 further including means biasing the male members (34) and lead-wire terminals (32) together at the intersections of the second elongate cavities (46) with the first elongate cavity (44).

4. The electrical brain-contact device (10) of claim 3 characterized in that:
- the lead-wire terminals (32) have substantially fixed cross-dimensions;
- the connector block (22,24) has a pair of adjacent second elongate cavities (46) corresponding to each lead-wire terminal (32); and
- each individual conductor (34) includes a pair of said male members (34) in the pair of second elongate cavities (46), said pair of male members (34) spaced from one another by an amount less than the fixed cross-dimension,
whereby the pair of male members (34) are forcibly spread in engaging the lead-wire terminals (32) therebetween to provide said biasing means.

5. The electrical brain-contact device (10) of claim 2 characterized in that the connector block (22,24) is substantially transparent, whereby engagement of the conductors (34) with the lead-wire terminals (32) is visible.

6. The electrical brain-contact device (10) of claim 2 characterized in that the connector block (22,24) is of non-conductive one-piece construction.

7. The electrical brain-contact device (10) of claim 2 characterized in that the connector block (22,24) includes a main surface (38) extending substantially parallel to the first elongate cavity (44), said second elongate cavities (46) extending from the main surface (38) to the first elongate cavity (44).

8. The electrical brain-contact device (10) of claim 7 characterized in that:
- the connector block (22,24) is formed of a plurality of sub-blocks (26) attached end-to-end by mating means (48) on each adjacent pair of sub-blocks (26);
- the sub-blocks (26) each forming a portion of the first elongate cavity (44) and a portion of the main surface (38) and having a subset of the second elongate cavities (46),
whereby the connector block (22,24) can be sized to accommodate varying numbers of lead-wire terminals (32) and conductors (34) by choosing the number of sub-blocks (26) to be attached to one another.

9. An electrical brain-contact device (10,110,112,113) having a tissue-engagement member (12,114,116) with a plurality of electrodes (30,120,126), separate lead wires (16,132) from the tissue-engagement member (12,114,116) for each electrode (30,120,126), and means (22,24,146,146a) away from the tissue-engagement member (12,114,116) to connect the lead wires (16,132) with individual conductors (34,170,170a),
characterized by:
- the lead wires (132) extending from the-tissue-engagement member (114,116) to a terminal mount (136) and to an array of lead-wire terminals (138) on and forming a part of the terminal mount (136), the terminal mount (136) including a sheathing (140) with the lead wires (132) therein and the lead-wire terminals (138) spaced therealong; and
- the connector means (146,146a) including a base member (148,148a) with a matching array of take-up terminals (170,170a), a yoke member (150,150a) movable with respect to the base member (148,148a) and forming therebetween a space (162,162a) for the terminal mount (136), and means (152,152a) to hold the yoke (150,150a) and base members (148,148a) together with the arrays in terminal-to-terminal contact,
whereby electrical connection of the brain-contact device (10,110,112,113) is facilitated.

10. The electrical brain-contact device (110,112,113) of claim 9 characterized in that:
- the terminal mount is an elongate member (136) having the lead-wire terminals (138) spaced therealong;
- the base member (148,148a) forms terminal niches (168,168a) along the terminal-mount space (162,162a);
- the take-up terminals (170,170a), when the terminal mount (136) is removed, extend from the niches (168,168a) into the terminal-mount space (162,162a); and
- the take-up terminals (170,170a) are depressed by the terminal mount (136), when the terminal mount (136) is in place, such that the lead-wire terminals (138) are in firm electrical contact with the take-up terminals (170,170a).

11. The electrical brain-contact device (10,110,112,113) of one of the foregoing claims characterized in that the sheathing (14,140) is tubular and the lead-wire terminals (32,138) are rings therearound.

12. The electrical brain-contact device (10,110,112,113) of one of the foregoing claims characterized in that the sheathing (14,140) extends from the tissue-engagement member (12,114,116) for substantially the entire length of the lead wires (16,132).

## Patentansprüche

1. Elektrische Gehirnkontaktvorrichtung (10, 110, 112, 113) mit einem gewebegekoppelten Glied (12, 114, 116), das viele Elektroden (30, 120 126) aufweist, mit getrennten Drahtadern (16, 132), die vom gewebegekoppelten Glied (12, 114, 116) wegführen und für jeweils eine Elektrode vorgesehen sind, und mit Mitteln (20, 22, 24, 146, 146a) außerhalb des gewebegekoppelten Glieds (12, 114, 116) zur Verbindung der Drahtadern (16, 132) mit individuellen Leitern (34, 170, 170a), **dadurch gekennzeichnet**, daß
- die Drahtadern (16) zu einer Drahtaderanschlußhalterung (18) und zu einer Anordnung aus Drahtaderanschlüssen (32) längs der Drahtaderanschlußhalterung führen, wobei die Drahtaderanschlußhalterung (18) eine die Drahtadern (16) aufnehmende Hülle (14) aufweist, längs der die Drahtaderanschlüsse (32) verteilt sind,
- die Verbindungsmittel einen Steckerträger (20) aufweisen, der die individuellen Stecker (34) in einer Steckeranordnung trägt, und
- ein Verbinderblock (22, 24) vorgesehen ist, der einen ersten Hohlraum (44) und zweite Hohlräume (46) aufweist, die den ersten Hohlraum (44) schneiden, wobei der erste Hohlraum (44) die Drahtaderanschlußhalterung (18) und die Anordnung der Zweiten Hohlräume die Steckeranordnung aufnimmt und wobei der Verbinderblock (22, 24), die Drahtaderanschlußhalterung (18) und die Steckeranordnung derart aufgebaut sind, daß die Drahtaderanschlüsse (32) und die individuellen Stecker (34) durch mechanische Kräfte miteinander in Kontakt gehalten werden.

2. Elektrische Gehirnkontaktvorrichtung (10) nach Anspruch 1, dadurch gekennzeichnet, daß
- die Drahtaderanschlußhalterung ein längliches Glied (18) ist, längs dem die Drahtaderanschlüsse (32) verteilt sind,
- der erste Hohlraum ein erster länglicher Hohlraum (44) im Verbinderblock (22, 24) ist, wobei dieser Hohlraum durch nichtleitendes Material gebildet wird und so geformt ist, daß er das längliche Glied (18) aufnimmt,
- die individuellen Steckerglieder Stecker (34) sind, die aus dem Steckerträger (20) herausragen, und
- die zweiten Hohlräume Zweite längliche Hohlräume (46) im Verbinderblock (22, 24) sind, wobei diese Hohlräume durch nichtleitendes Material gebildet werden und so geformt sind, daß sie die Steckerglieder (34) aufnehmen.

3. Elektrische Gehirnkontaktvorrichtung (10) nach Anspruch 2, dadurch gekennzeichnet, daß Mittel zum Aneinanderdrücken der Steckerglieder (34) an die Drahtaderanschlüsse (32) an den Schnittstellen der zweiten länglichen Hohlräume (46) mit dem ersten länglichen Hohlraum (44) vorgesehen sind.

4. Elektrische Gehirnkontaktvorrichtung (10) nach Anspruch 3, dadurch gekennzeichnet, daß
- die Drahtaderanschlüsse (32) einen festgelegten Durchmesser haben,
- der Verbinderblock (22, 24) Paare benachbarter, zweiter, länglicher Hohlräume (46) aufweist, von denen jedes Paar einem Drahtaderanschluß (32) zugeordnet ist,
- jedes individuelle Steckerglied (34) ein Paar der Steckerglieder (34) zum Einstecken in das Paar der zweiten länglichen Hohlräume (46) aufweist, wobei die Stecker jedes Steckerpaars voneinander einen Abstand aufweisen, der geringer als der festgelegte Durchmesser der Drahtaderanschlüsse ist und
- jedes Paar der Steckerglieder (34) heim Eingriff mit dem zwischen sich befindlichen Drahtaderanschluß (32) kräftig gequetscht und dadurch das Druckmittel geschaffen wird.

5. Elektrische Gehirnkontaktvorrichtung (10) nach Anspruch 2, dadurch gekennzeichnet, daß der Verbinderblock (22, 24) im wesentlichen durchsichtig ausgebildet ist, wobei die Verbindung der Stecker (34) mit den Drahtaderanschlüssen (32) sichtbar ist.

6. Elektrische Gehirnkontaktvorrichtung (10) nach Anspruch 2, dadurch gekennzeichnet, daß der Verbinderblock (22, 24) aus nichtleitenden Material besteht und einstückig ausgebildet ist.

7. Elektrische Gehirnkontaktvorrichtung (10) nach Anspruch 2, dadurch gekennzeichnet, daß der Verbinderblock (22, 24) eine Hauptfläche (38) aufweist, die im wesemtlichen parallel zum ersten länglichen Hohlraum (44) verläuft, wobei die zweiten länglichen Hohlräume (46) von der Hauptfläche (38) zum ersten länglichen Hohlraum (44) führen.

8. Elektrische Gehirnkontaktvorrichtung (10) nach Anspruch 7, dadurch gekennzeichnet, daß
- der Verbinderblock (22, 24) durch eine Anzahl Teilblöcke (26) gebildet wird, die an benachbarten Enden mittels Passungen (48) miteinander verbunden sind,
- jeder Teilblock (26) einen Teil des ersten länglichen Hohlraums (44), einen Teil der Hauptfläche (38) und eine Untermenge der zweiten länglichen Hohlräume (46) aufweist und
- dabei der Verbinderblock (22, 24) so bemessen werden kann, daß verschiedene Anzahlen der Drahtaderanschlüsse (32) an die Stecker (34) durch Wahl der Anzahl der aneinander zu befestigenden Teilblöcke (26) angepaßt werden.

9. Elektrische Gehirnkontaktvorrichtung (10, 110, 112, 113) mit einem gewebegekoppelten Glied (12, 114, 116), das viele Elektroden (30, 120 126) aufweist, mit getrennten Drahtadern (16, 132), die vom gewebegekoppelten Glied (12, 114, 116) wegführen und für jeweils eine Elektrode vorgesehen sind, und mit Mitteln (20, 22, 24, 146, 146a) außerhalb des gewebegekoppelten Glieds (12, 114, 116) zur Verbindung der Drahtadern (16, 132) mit individuellen Leitern (34, 170, 170a), **dadurch gekennzeichnet**, daß
- die Drahtadern (132, Fig. 9) vom gewebegekoppelten Glied (114, 116 zu einer Drahtaderanschlußhalterung (136) und zu einer Anordnung aus Drahtaderanschlüssen (138) führen, die auf einem Teil der Drahtaderanschlußhalterung (136) angebracht ist und einen Teil der Drahtaderanschlußhalterung bildet, wobei die Drahtaderanschlußhalterung (136) eine Hülle (140) aufweist, in der die Drahtadern (132) geführt sind und auf der die Drahtaderanschlüsse (138) verteilt sind, und
- die Verbindungsmittel (146, 146a) ein Basisglied (148, 148a) mit einer Anpassungsanordnung aus Aufnahmeanschlüssen (170, 170a), ein Jochglied (150, 150a), das inbezug auf das Basisglied (148, 148a) beweglich ist und zusammen mit diesem Basisglied einen Raum für die Aufnahme der Drahtaderanschlußhalterung (136) bildet, und Mittel (152, 152a) zur Zusammenhaltung des Jochglieds (150, 150a) und des Basisglieds (148, 148a) umfaßt, wobei die Anschlüsse der Anordnungen in Kontakt kommen,
wobei die elektrische Verbindung der Gehirnkontaktvorrichtung (10, 110, 112, 113) erleichtert wird.

10. Elektrische Gehirnkontaktvorrichtung (110, 112, 113) nach Anspruch 9, dadurch gekennzeichnet, daß
- die Drahtaderanschlußhalterung ein längliches Glied (136) ist, auf dem die Drahtaderanschlüsse (138) verteilt sind,
- das Basisglied (148, 148a) Anschlußauskehlungen (168, 168a) längs des Anschlußhalterungsraums (162, 162a) aufweist,
- die Aufnahmeanschlüsse (170, 170a) bei der Entfernung der Anschlußhalterung (136) von den Auskehlungen (168, 168a) in den Anschlußhalterungsraum (162, 162a) ragen und
- die Aufnahmeanschlüsse (170, 170a) durch die Anschlußhalterung (136) zusammengedrückt werden, wenn die Anschlußhalterung (136) eingesetzt ist, derart, daß die Drahtaderanschlüsse (138) in festen elektrischen Kontakt mit den Aufnahmeanschlüssen (170, 170a) kommen.

11. Elektrische Gehirnkontaktvorrichtung (10, 110, 112, 113) nach einem der vorstehenden Ansprüche, dadurch gekennzeichnet, daß die Hülle (14, 140) rohrförmig ausgebildet ist und daß die Drahtaderanschlüsse (32, 138) als Ringe um diese Hülle ausgebildet sind.

12. Elektrische Gehirnkontaktvorrichtung (10, 110, 112, 113) nach einem der vorstehenden Ansprüche, dadurch gekennzeichnet, daß die Hülle (14, 140), die vom gewebegekoppelten Glied (12, 114, 116) kommt, im wesentlichen über die ganze Länge der Drahtadern (16, 132) diese umhüllt.

## Revendications

**1.** Dispositif électrique (10, 110, 112, 113) en contact avec le cerveau comportant un organe (12, 114, 116) en engagement avec le tissu, organe muni d'une pluralité d'électrodes (30, 120, 126), des fils conducteurs sépares (16, 132) issus de l'organe (12, 114, 116) en engagement avec le tissu pour chaque électrode (30, 120, 126), et des moyens (20, 22, 24, 146, 146a) éloignés de l'organe (12, 114, 116) en engagement avec le tissu pour connecter les fils conducteurs (16, 132) à des conducteurs individuels (34, 170, 170a), caractérisé en ce que
- les fils conducteurs (16) s'étendent jusqu'à un support à bornes (18) et à une rangée de bornes pour fils conducteurs (32) sur un support à bornes (18) et constituant une partie de celle-ci, le support à bornes (18) comprenant une enveloppe (14) dans laquelle se trouvent les fils conducteurs (16) et les extrémités (32) des fils conducteurs étant espacés le long de l'enveloppe;
- les moyens connecteurs comprennent un support de conducteur (20) maintenant les conducteurs individuels (34) dans une rangée de conducteurs; et
- il comporte un bloc connecteur (22, 24) présentant une première cavité (44) et une rangée de secondes cavités (46) dont chacune fait intersection avec la première cavité (44), la première cavité (44) recevant le support à bornes (18) et la rangée de secondes cavités (46) recevant la rangée de conducteurs, le bloc connecteur (22, 24), le support à bornes (18) et la rangée de conducteurs étant disposés de manière telle que les extrémités (32) des fils conducteurs et les conducteurs individuels (34) sont maintenus en engagement par interaction mécanique.

**2.** Dispositif électrique (10) en contact avec le cerveau suivant la revendication 1, caractérisé en ce que
- le support à bornes est un organe allongé (18) sur lequel sont disposées avec écartement les extrémités (32) des fils conducteurs;
- la première cavité est une cavité allongée (44) dans le bloc connecteur (22, 24) réalisé en un matériau non conducteur et présentant une forme apte à recevoir l'organe allongé (18);
- les conducteurs individuels sont des organes mâles (34) faisant protubérance depuis le support (20) de conducteurs; et
- les secondes cavités sont des cavités allongées (46) dans le bloc connecteur (22, 24) réalisé en un matériau non conducteur et présentant une forme apte à recevoir les organes mâles (34).

**3.** Dispositif électrique (10) en contact avec le cerveau suivant la revendication 2 comprenant en outre des moyens sollicitant les organes mâles (34) et les extrémités des fils conducteurs (32) pour qu'ils viennent en contact aux intersections des secondes cavités allongées (46) avec la première cavité allongée (44).

**4.** Dispositif électrique (10) en contact avec le cerveau suivant la revendication 3 caractérisé en ce que
- les extrémités (32) ont des sections transversales en substance fixes;
- le bloc connecteur (22, 24) présente une paire de secondes cavités allongées adjacentes (46) correspondant à chaque extrémité (32) de fil conducteur; et
- chaque conducteur individuel (34) comprend une paire desdits organes mâles (34) dans la paire de secondes cavités allongées (46), les organes mâles (34) de ladite paire étant espacés l'un de l'autre d'une valeur moindre que la section transversale fixe,
où la paire d'organes mâles (34) est écartée sous l'action d'une force pour venir en contact avec les extrémités de fils conducteurs (32), constituant de la sorte ledit moyen de sollicitation.

**5.** Dispositif électrique (10) en contact avec le cerveau suivant la revendication 2, caractérisé en ce que le bloc connecteur (22, 24) est en substance transparent, le contact des conducteurs (34) avec les extrémités des fils conducteurs (32) étant de la sorte visible.

**6.** Dispositif électrique (10) en contact avec le cerveau suivant la revendication 2, caractérisé en ce que le bloc connecteur (22, 24) est fabriqué en une pièce en un matériau non conducteur.

**7.** Dispositif électrique (10) en contact avec le cerveau suivant la revendication 2, caractérisé en ce que le bloc connecteur (22, 24) comporte une surface principale (38) orientée en substance parallèlement à la première cavité allongée (44), lesdites secondes cavités allongées (46) s'étendant depuis la surface principale (38) jusqu'à la première cavité allongée.

**8.** Dispositif électrique (10) en contact avec le cerveau suivant la revendication 7, caractérisé en ce que le bloc connecteur (22, 24) comprend une surface principale (38) qui s'étend en substance parallèlement à la première cavité allongée (44), lesdites secondes cavités allongées (46) s'étendant depuis la surface principale (38) jusqu'à la première cavité allongée (44).

**8.** Dispositif électrique (10) en contact avec le cerveau suivant la revendication 7, caractérisé en ce que:
- le bloc connecteur (22, 24) est formé d'une pluralité de sous-blocs, (26) réunis bout à bout par des moyens assortis (48) sur chaque paire adjacente de sous-blocs (26);
- les sous-blocs constituent chacun une portion de la première cavité allongée (44) et une portion de la surface principale (38) et présentant un sous-ensemble de secondes cavités allongées (46),
dans lequel le bloc connecteur (22, 24) peut être dimensionné de manière à recevoir un nombre variable d'extrémités de fils conducteurs (32) et de conducteurs (34) en fonction du nombre de sous-blocs (26) qui doivent être réunis.

**9.** Dispositif électrique (10, 110, 112, 113) en contact avec le cerveau comportant un organe (12, 114, 116) en engagement avec le tissu, organe muni d'une pluralité d'électrodes (30, 120, 126), des fils conducteurs séparés (16, 132) issus de l'organe (12, 114, 116) en engagement avec le tissu pour chaque électrode (30, 120, 126), et des moyens (20, 22, 24, 146, 146a) éloignés de l'organe (12, 114, 116) en engagement avec le tissu pour connecter les fils conducteurs (16, 132) à des conducteurs individuels (34, 170, 170a), caractérisé en ce que
- les fils conducteurs (132) s'étendent depuis l'organe (114, 116) en contact avec le tissu jusqu'à un support (136) pour extrémités de fils conducteurs et jusqu'à un ensemble d'extrémités de fils conducteurs (138) sur le support (136) et faisant partie intégrante de celui-ci, le support (136) comprenant un gainage (140) dans lequel sont guidés les fils conducteurs et le long duquel sont espacés les extrémités des fils conducteurs (138); et
- les moyens connecteurs (146, 146a) comprennent un élément de base avec une disposition d'adaptation constituée par des fiches réceptrices (170, 170a), un élément de culasse (150, 150a) mobile par rapport l'élément de base (148, 148a et qui conjointement avec cet élément de base constitue une cavité (162, 162a) pour la réception du support (136) des extrémités, et des moyens (152, 152a) pour réunir l'élément de culasse (150, 150a) et l'élément de base (148, 148a) de manière à ce que les extrémités des dispositions d'adaptation viennent en contact électrique,
ce qui facilite la connexion électrique du dispositif de contact (10, 110, 112, 113) avec le cerveau.

**10.** Dispositif électrique (110, 112, 113) en contact avec le cerveau suivant la revendication 9, caractérisé en ce que:
- le support pour extrémités est un organe allongé (136) le long duquel sont distribuées les extrémités (138) des fils conducteurs;
- le long de la cavité (162, 162a) pour la réception du support des extrémités, l'élément de base (148, 148a) présente des niches (168, 168a) pour la réception des extrémités;
- lorsque le support (136) pour les extrémités est déposé, les fiches (170, 170a) pour extrémités s'étendent depuis les niches (168, 168a) jusque l'intérieur de la cavité (162, 162a) pour la réception du support des extrémités;
- les fiches (170, 170a) pour extrémités sont comprimées par le support (136) pour les extrémités, lorsque le support (136) pour les extrémités est mis en place, de manière telle que les extrémités (138) des fils conducteurs sont en contact électrique ferme avec les fiches (170, 170a) pour extrémités.

**11.** Dispositif électrique (10, 110, 112, 113) en contact avec le cerveau suivant l'une quelconque des revendications précédentes, caractérisé en ce que l'enveloppe (14, 140) est tubulaire et en ce que les extrémités (32, 138) sont disposées sous forme d'anneaux autour de cette enveloppe.

**12.** Dispositif électrique (10, 110, 112, 113) en contact avec le cerveau suivant l'une quelconque des revendications précédentes, caractérisé en ce que l'enveloppe (14, 140) s'étend depuis l'organe (12, 114, 116) en contact avec le tissus sur en substance toute la longueur des fils conducteurs (16, 132).
